(19) Europäisches Patentamt
European Patent Office
Office européen des brevets

(11) EP 4 541 929 A1

# (12) EUROPEAN PATENT APPLICATION

published in accordance with Art. 153(4) EPC

(43) Date of publication:
**23.04.2025 Bulletin 2025/17**

(21) Application number: **23826942.7**

(22) Date of filing: **01.06.2023**

(51) International Patent Classification (IPC):
***C23C 16/18*** *(2006.01)* ***C07C 31/38*** *(2006.01)*
***C07F 1/02*** *(2006.01)* ***C23C 16/455*** *(2006.01)*

(52) Cooperative Patent Classification (CPC):
**C07C 31/38; C07F 1/02; C23C 16/18; C23C 16/455**

(86) International application number:
**PCT/JP2023/020483**

(87) International publication number:
**WO 2023/248755 (28.12.2023 Gazette 2023/52)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **20.06.2022 JP 2022098679**

(71) Applicant: **L'AIR LIQUIDE, SOCIETE ANONYME POUR L'ETUDE ET L'EXPLOITATION DES PROCEDES GEORGES CLAUDE**
**75007 Paris (FR)**

(72) Inventors:
- **KAMIMURA, Sunao**
  **Yokosuka-shi, Kanagawa 239-0847 (JP)**
- **GREER, Jamie**
  **Yokosuka-shi, Kanagawa 239-0847 (JP)**
- **DUSSARRAT, Christian**
  **Yokosuka-shi, Kanagawa 239-0847 (JP)**

(74) Representative: **Air Liquide**
**L'Air Liquide S.A.**
**Direction de la Propriété Intellectuelle**
**75, Quai d'Orsay**
**75321 Paris Cedex 07 (FR)**

## (54) MATERIAL FOR CHEMICAL VAPOR DEPOSITION, FILM PRODUCTION METHOD, AND FILM

(57) The present invention provides a material for chemical vapor deposition, a film production method, and a film which are novel. Provided is a material for chemical vapor deposition represented by formula (1). (In the formula, E is an oxygen atom, a sulfur atom, a selenium atom, or a tellurium atom. R1, R2, and R3, are each independently a hydrogen atom or a linear, branched, or cyclic C1-7 organic group, and at least one of R1, R2, and R3 includes a fluorine atom. A case where R1, R2, and R3 are all a hydrogen atom is excluded.)

EP 4 541 929 A1

## Description

Technical field

The present disclosure relates to a chemical vapor deposition material, film production method, and film.

Prior art

In batteries, durability and high ion conductivity are required. For example, in lithium ion batteries it is necessary to maintain electrode durability and to have a high ion conductivity. With regard to durability, the provision of a film that covers the positive electrode and the negative electrode in lithium ion batteries is known. Lithium phosphate oxynitride (LiPON), lithium niobate ($LiNbO_3$), lithium titanate ($Li_2TiO_3$), etc., are examples of compounds contained in the film. Patent document 1 discloses a chemical vapor deposition material for producing lithium phosphate oxynitride layers.

Prior art documents

Patent documents

Patent document 1: Japanese unexamined patent 2017-48445

Summary of the invention

Problem to be solved by the invention

The aim of the present disclosure is the provision of a novel chemical vapor deposition material, film production method, and film.

Means of solving the problem

The present inventors discovered that the abovementioned problem can be solved by a chemical vapor deposition material represented by formula (1) below.

[1] Chemical vapor deposition material represented by formula (1) below.

[Chemical notation 1]

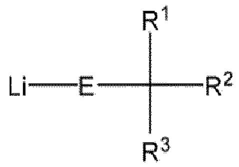

(1)

(Here, E is an oxygen atom, sulfur atom, selenium atom or tellurium atom.
$R^1$, $R^2$ and $R^3$ are each independently a hydrogen atom or a C1-7 straight-chain, branched or cyclic organic group, and at least one of $R^1$, $R^2$ and $R^3$ contains a fluorine atom.
Also, $R^1$, $R^2$ and $R^3$ are not all hydrogen atoms.)

[2] The chemical vapor deposition material described in [1], where $R^1$, $R^2$ and $R^3$ are each independently a hydrogen atom or a C1-5 fluorinated alkyl group, C2-5 alkenyl group, C2-5 fluorinated alkenyl group or C1-5 fluorinated alkoxyalkyl group.
[3] The chemical vapor deposition material described in [1] or [2], where at least one of $R^1$, $R^2$ and $R^3$ is $-CF_3$, $-CF_2CF_3$, $-CF_2CF_2CF_3$, $-(CH_2CH=CH_2)$ or $-(CH_2OCH_2CF_3)$.
[4] The chemical vapor deposition material described in any of [1] to [3], where $R^1$ is $-CF_3$.
[5] The chemical vapor deposition material described in any of [1] to [4], where at least one of $R^2$ and $R^3$ is a hydrogen atom.
[6] The chemical vapor deposition material described in any of [1] to [5], where E is an oxygen atom.
[7] A film production method provided with a formation step whereby the chemical vapor deposition material described

in any of [1] to [6] is vapor-deposited onto a substrate to form a film.

[8] The film production method described [7], where
said formation step proceeds in the presence of at least one coreactant chosen from the group consisting of $O_2$, $O_3$, $H_2O$, $H_2O_2$, NO, $NO_2$, $N_2O$, $PH_3$ and TMPO.

[9] The film production method described in [7] or [8], where
the temperature of the reactor in said formation step is ≥150°C to ≤350°C.

[10] The film production method described in any of [7] to [9], where

said film contains a compound represented by $Li_xO_yF_z$
wherein

$$2.6 \leq x \leq 4.3,$$

$$1.8 \leq y \leq 2.3$$

and

$$4.3 \leq z \leq 6.6.$$

[11] The film production method described in any of [7] to [10], where the reflection coefficient of said film is ≥1.1 to ≤1.5.

[12] Film that contains a compound represented by $Li_xO_yF_z$
wherein

$$2.6 \leq x \leq 4.3,$$

$$1.8 \leq y \leq 2.3$$

and

$$4.3 \leq z \leq 6.6.$$

[13] The film described in [12], where
the reflection coefficient of said film is ≥1.1 to ≤1.5.

Advantages of the invention

The present disclosure allows the provision of a novel chemical vapor deposition material, film production method, and film.

Embodiments of the invention

Embodiments of the present disclosure are described below, but the present disclosure is not particularly limited to these embodiments, and can be implemented with appropriate modifications. Also, any of the various configurations described below can be implemented in combination.

Terminology definitions

Standard abbreviations for elements from the periodic table of elements are used in the present specification. Therefore, elements are represented by these abbreviations. For example, Li denotes lithium, O denotes oxygen, S denotes sulfur, Se denotes selenium, Te denotes tellurium, H denotes hydrogen, C denotes carbon, and F denotes fluorine. The same applies to other elements.

In the present specification, CVD (chemical vapor deposition) denotes the chemical gaseous phase deposition method or the chemical vapor deposition method, and ALD (atomic layer deposition) denotes the atomic layer deposition method. It should be noted that ALD is a type of CVD.

In the present specification, an "organic group" is a group containing at least 1 carbon atom.

In the present specification, a "fluorinated alkyl group" is an alkyl group (functional group) wherein one or more of

its hydrogen atoms are substituted by fluorine. Alkyl groups are represented by the general formula $C_nH_{2n+1}$ (where n is ≥1). "Fluorinated alkyl group" means the same as fluoroalkyl group.

In the present specification, a "fluorinated alkenyl group" is an alkenyl group (functional group) wherein one or more of its hydrogen atoms are substituted by fluorine. Alkenyl groups are represented by the general formula $C_nH_{2n-1}$ (where n is ≥2).

In the present specification, a "fluorinated alkoxyalkyl group" is an alkoxyalkyl group (functional group) wherein one or more of its hydrogen atoms are substituted by fluorine. Alkoxy groups are represented by the general formula $C_nH_{2n+1}O$ (where n is an integer ≥1), and alkoxyalkyl groups are represented by the general formula $C_nH_{2n+1}O\text{-}C_mH_{2m}$ (where n and m are each independently an integer ≥1).

In the present specification, "TMPO" is trimethyl phosphate.

When used in the present specification, the abbreviation "Me" denotes methyl group, the abbreviation "Et" denotes ethyl group; the abbreviation "Pr" denotes propyl group; the abbreviation ""Pr" denotes "normal" or straight-chain propyl group; the abbreviation "$^i$Pr" denotes isopropyl group; the abbreviation "Bu" denotes butyl group; the abbreviation "$^n$Bu" denotes "normal" or straight-chain butyl group; the abbreviation "$^t$Bu" denotes tert-butyl group, also known as 1,1-dimethylethyl; the abbreviation "$^s$Bu" denotes sec-butyl group, also known as 1-methylpropyl; and the abbreviation "$^i$Bu" denotes isobutyl group, also known as 2-methylpropyl.

<u>Chemical vapor deposition material</u>

The chemical vapor deposition material relating to the present embodiments is represented by formula (1) below.

[Chemical notation 2]

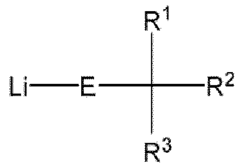

(1)

(Here, E is an oxygen atom, sulfur atom, selenium atom or tellurium atom.
$R^1$, $R^2$ and $R^3$ are each independently a hydrogen atom or a C1-7 straight-chain, branched or cyclic organic group, and at least one of $R^1$, $R^2$ and $R^3$ contains a fluorine atom.
Also, $R^1$, $R^2$ and $R^3$ are not all hydrogen atoms.)

This type of chemical vapor deposition material allows the provision of a novel chemical vapor deposition material.

<u>Metal</u>

The chemical vapor deposition material has Li (lithium) as its metal element.

<u>Atom E</u>

Atom E belongs to group 16 of the periodic table. For example, it is an oxygen atom, sulfur atom, selenium atom or tellurium atom. Atom E is preferably an oxygen atom.

<u>Substituent groups $R^1$, $R^2$ and $R^3$</u>

$R^1$, $R^2$ and $R^3$ are each independently a hydrogen atom or a C1-7 straight-chain, branched or cyclic organic group. And at least one of $R^1$, $R^2$ and $R^3$ contains a fluorine atom. Also, $R^1$, $R^2$ and $R^3$ are not all hydrogen atoms.

Examples of C1-7 straight-chain organic groups are methyl group, ethyl group, propyl group, butyl group, pentyl group, hexyl group, heptyl group, ethenyl group, propenyl group, etc.

Examples of C1-7 branched organic groups are tert-butyl group, sec-butyl group and isobutyl group.

Examples of C1-7 cyclic organic groups are cyclic alkyl groups, alkenyl groups and alkynyl groups.

The abovementioned C1-7 organic groups may have a divalent heteroatom-containing group between a carbon-carbon bond, and some or all of the hydrogen atoms of the abovementioned organic groups may be substituted by monovalent heteroatom-containing groups.

Examples of heteroatoms in the divalent or monovalent heteroatom-containing groups are oxygen atom, nitrogen atom, sulfur atom, phosphorus atom, silicon atom, halogen atom, etc. Examples of halogen atoms are fluorine atom, chlorine atom, bromine atom and iodine atom.

Examples of divalent heteroatom-containing groups are -CO-, -CS-, -NH-, -O-, -S-, - $SO_2$- and divalent groups containing combinations of these.

Examples of monovalent heteroatom-containing groups are hydroxy group, carboxy group, sulfanyl group, cyano group, nitro group, halogen atoms, etc.

Preferably, $R^1$, $R^2$ and $R^3$ are each independently a hydrogen atom or a C1-5 fluorinated alkyl group, C2-5 alkenyl group, C2-5 fluorinated alkenyl group or C2-5 fluorinated alkoxyalkyl group.

More preferably, at least one of $R^1$, $R^2$ and $R^3$ is $-CF_3$, $-CF_2CF_3$, $-CF_2CF_2CF_3$, $-(CH_2CH=CH_2)$ or $-(CH_2OCH_2CF_3)$.

Even more preferably, R1 is $-CF_3$.

Particularly preferably, at least one of $R^2$ and $R^3$ is a hydrogen atom.

Specific examples of chemical vapor deposition materials

Below are examples of chemical vapor deposition materials.

$Li\text{-}OCH_2CF_3$, $Li\text{-}OCH_2CF_2CF_3$, $Li\text{-}OC(CH_2CH=CH_2)(CF_3)_2$, $Li\text{-}OC(CF_3)_3$, $Li\text{-}OCH(CF_3)_2$, $Li\text{-}O(CH_2)_2OCH_2CF_3$, $Li\text{-}OC(CH_3)(CF_3)_2$, $Li\text{-}OCH(CH_2OCH_2CF_3)_2$

[Chemical notation 3]

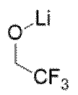

**$Li\text{-}OCH_2CF_3$**

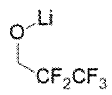

**$Li\text{-}OCH_2CF_2CF_3$**

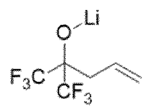

**$Li\text{-}OC(CH_2CH=CH_2)(CF_3)_2$**

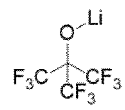

**$Li\text{-}OC(CF_3)_3$**

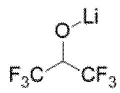

**$Li\text{-}OCH(CF_3)_2$**

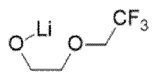

**$Li\text{-}O(CH_2)_2OCH_2CF_3$**

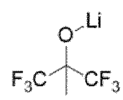

**$Li\text{-}OC(CH_3)(CF_3)_2$**

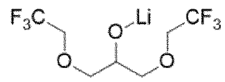

**$Li\text{-}OCH(CH_2OCH_2CF_3)_2$**

Below are more examples of chemical vapor deposition materials.

$LiOC(Et)_2(CF_3)$, $LiOC(Pr)_2(CF_3)$, $LiOC(^{n}Bu)_2(CF_3)$, $LiOC(^{i}Bu)_2(CF_3)$, $LiOC(^{t}Bu)_2(CF_3)$, $LiOC(^{s}Bu)_2(CF_3)$, $LiOC(MeEt)(CF_3)$, $LiOC(MePr)(CF_3)$, $LiOC(Me^{n}Bu)(CF_3)$, $LiOC(Me^{i}Bu)(CF_3)$, $LiOC(Me^{t}Bu)(CF_3)$, $LiOC(Me^{s}Bu)(CF_3)$, $LiOCH_2(CF_2CF_3)$, $LiOCH(CF_2CF_3)_2$, $LiOC(CH_3)_2(CF_2CF_3)$, $LiOC(MeEt)(CF_2CF_3)LiOC(MePr)(CF_2CF_3)$, $LiOC(Me^{i}Bu)(CF_2CF_3)$, $LiOC(MenBu)(CF_2CF_3)$, $LiOC(MetBu)(CF_2CF_3)$, $LiOC(Me^{s}Bu)(CF_2CF_3)$, $LiOC(CH_3)(CF_2CF_3)_2$, $LiOC(CF_2CF_3)_3$, $LiOC(Et)(CF_3)_2$, $LiOC(^{n}Pr)(CF_3)_2$, $LiOC(^{i}Pr)(CF_3)_2$, $LiOC(^{n}Bu)(CF_3)_2$, $LiOC(^{t}Bu)(CF_3)_2$, $LiOC(^{s}Bu)(CF_3)_2$, $LiOC(^{i}Bu)(CF_3)_2$, $LiO(CH_2)_2OCH_2CF_3$, $LiO(CH_2)_2OCH(CF_3)_2$, $LiO(CH_2)_2OC(CF_3)_3$, $LiO(CH_2)_2OC(CH_3)(CF_3)_2$, $LiO(CH_2)_2OC(CH_3)_2(CF_3)$, $LiO(CH_2)_2OC(Et)_2(CF_3)$, $LiO(CH_2)_2OC(Pr)_2(CF_3)$, $LiO(CH_2)_2OC(^{n}Bu)_2(CF_3)$, $LiO(CH_2)_2OC(^{i}Bu)_2(CF_3)$, $LiO(CH_2)_2OC(^{t}Bu)_2(CF_3)$, $LiO(CH_2)_2OC(^{s}Bu)_2(CF_3)$, $LiO(CH_2)_2OC(MeEt)(CF_3)$, $LiO(CH_2)_2OC(MePr)(CF_3)$, $LiO(CH_2)_2OC(Me^{n}Bu)(CF_3)$, $LiO(CH_2)_2OC(MeiBu)(CF_3)$, $LiO(CH_2)_2OC(MetBu)(CF_3)$, $LiO(CH_2)_2OC(Me^{s}Bu)(CF_3)$, $LiO(CH_2)_2OCH_2(CF_2CF_3)$, $LiO(CH_2)_2OCH(CF_2CF_3)_2$, $LiO(CH_2)_2OC(CH_3)_2(CF_2CF_3)$, $LiO(CH_2)_2OC(MeEt)(CF_2CF_3)$, $LiO(CH_2)_2OC(MePr)(CF_2CF_3)$, $LiO(CH_2)_2OC(Me^{i}Bu)(CF_2CF_3)$,

$LiO(CH_2)_2OC(Me^{n}Bu)(CF_2CF_3)$, $LiO(CH_2)_2OC(Me^{t}Bu)(CF_2CF_3)$, $LiO(CH_2)2OC(Me^{s}Bu)(CF_2CF_3)$, $LiO(CH_2)_2OC(CH_3)(CF_2CF_3)_2$, $LiO(CH_2)_2OC(CF_2CF_3)_3$, $LiO(CH_2)_2OC(CH_2CH=CH_2)(CF_3)_2$, $LiO(CH_2)_2OC(Et)(CF_3)_2$, $LiO(CH_2)_2OC(^{n}Pr)(CF_3)_2$, $LiO(CH_2)_2OC(^{i}Pr)(CF_3)_2$, $LiO(CH_2)_2OC(^{n}Bu)(CF_3)_2$, $LiO(CH_2)_2OC(^{t}Bu)(CF_3)_2$, $LiO(CH_2)_2OC(^{s}Bu)(CF_3)_2$, $LiO(CH_2)_2OC(^{i}Bu)(CF_3)_2$, $LiOCH(Me)CH_2OCH_2CF_3$, $LiOCH(Me)CH_2OCH(CF_3)_2$, $LiOCH(Me)CH_2OC(CF_3)_3$, $LiOCH(Me)CH_2OC(CH3)(CF_3)_2$, $LiOCH(Me)CH_2OC(CH_3)_2(CF_3)$, $LiOCH(Me)CH_2OC(Et)_2(CF_3)$, $LiOCH(Me)CH_2OC(Pr)_2(CF_3)$, $LiOCH(Me)CH_2OC(^{n}Bu)_2(CF_3)$, $LiOCH(Me)CH_2OC(^{i}Bu)_2(CF_3)$, $LiOCH(Me)CH_2OC(^{t}Bu)_2(CF_3)$, $LiOCH(Me)CH_2OC(^{s}Bu)_2(CF_3)$, $LiOCH(Me)CH_2OC(MeEt)(CF_3)$, $LiOCH(Me)CH_2OC(MePr)(CF_3)$, $LiOCH(Me)CH_2OC(Me^{n}Bu)(CF_3)$, $LiOCH(Me)CH_2OC(Me^{i}Bu)(CF_3)$, $LiOCH(Me)CH_2OC(Me^{t}Bu)(CF_3)$, $LiOCH(Me)CH_2OC(Me^{s}Bu)(CF_3)$, $LiOCH(Me)CH_2OCH_2(CF_2CF_3)$, $LiOCH(Me)CH_2OCH(CF_2CF_3)_2$, $LiOCH(Me)CH_2OC(CH_3)_2(CF_2CF_3)$, $LiOCH(Me)CH_2OC(MeEt)(CF_2CF_3)$, $LiOCH(Me)CH_2OC(MePr)(CF_2CF_3)$, $LiOCH(Me)CH_2OC(Me^{i}Bu)(CF_2CF_3)$, $LiOCH(Me)CH_2OC(MenBu)(CF_2CF_3)$, $LiOCH(Me)CH_2OC(Me^{t}Bu)(CF_2CF_3)$, $LiOCH(Me)CH_2OC(Me^{s}Bu)(CF_2CF_3)$, $LiOCH(Me)CH_2OC(CH_3)(CF_2CF_3)_2$, $LiOCH(Me)CH_2OC(CF_2CF_3)_3$, $LiOCH(Me)CH_2OC(CH_2CH=CH_2)(CF_3)_2$,

LiOCH(Me)CH$_2$OC(Et)(CF$_3$)$_2$, LiOCH(Me)CH $_2$OC($^n$Pr)(CF$_3$)$_2$, LiOCH(Me)CH$_2$OC($^i$Pr)(CF$_3$)$_2$, L iOCH (Me) CH$_2$OC($^n$Bu)(CF$_3$)2, LiOCH(Me)CH$_2$OC ($^t$Bu)(CF$_3$)$_2$, LiOCH(Me)CH$_2$OC($^s$Bu)(CF$_3$)$_2$, Li OCH (Me) CH$_2$OC($^i$Bu)(CF$_3$)$_2$, LiOCH$_2$CH(Me)OCH$_2$ CF$_3$, LiOCH$_2$CH(Me)OCH(CF$_3$)$_2$, LiOCH$_2$CH(Me)O C (CF$_3$)$_3$, LiOCH$_2$CH(Me)OC(CH$_3$)(CF$_3$)$_2$, LiOCH$_2$ CH(Me)OC(CH$_3$)$_2$(CF$_3$), LiOCH$_2$CH(Me)OC(Et)$_2$ (CF$_3$), LiOCH$_2$CH(Me)OC(Pr)$_2$(CF$_3$), LiOCH$_2$CH (Me) OC($^n$Bu)$_2$(CF$_3$), LiOCH$_2$CH(Me)OC($^i$Bu)$_2$(C F$_3$), LiOCH$_2$CH(Me)OC($^t$Bu)$_2$(CF$_3$), LiOCH$_2$CH(M e)OC($^s$Bu)$_2$(CF$_3$), LiOCH$_2$CH(Me)OC(MeEt)(CF $_3$), LiOCH$_2$CH(Me)OC(MePr)(CF$_3$), LiOCH$_2$CH(M e)OC(Me$^n$Bu)(CF$_3$), LiOCH$_2$CH(Me) OC(Me$^i$Bu) (CF$_3$), LiOCH$_2$CH(Me)OC(MetBu)(CF$_3$), LiOCH$_2$ CH(Me)OC(Me$^s$Bu)(CF$_3$), LiOCH$_2$CH(Me)OCH$_2$(C F$_2$CF$_3$), LiOCH$_2$CH(Me)OCH(CF$_2$CF$_3$)$_2$, LiOCH$_2$CH (Me)OC(CH$_3$)$_2$(CF$_2$CF$_3$), LiOCH$_2$CH(Me)OC(MeE t)(CF$_2$CF$_3$), LiOCH$_2$CH(Me)OC(MePr)(CF$_2$CF $_3$), LiOCH$_2$CH(Me)OC(Me$^i$Bu)(CF$_2$CF$_3$), LiOCH$_2$C H(Me)OC(Me$^n$Bu)(CF$_2$CF$_3$), LiOCH$_2$CH(Me)OC(M e$^t$Bu)(CF$_2$CF$_3$), LiOCH$_2$CH(Me)OC(Me$^s$Bu)(CF$_2$C F$_3$), LiOCH$_2$CH(Me)OOC(CH$_3$)(CF$_2$CF$_3$)$_2$, LiOCH$_2$ CH(Me)OC(CF$_2$CF$_3$)$_3$, LiOCH$_2$CH(Me)OC(CH$_2$CH= CH$_2$)(CF$_3$)$_2$, LiOCH$_2$CH(Me)OC(Et)(CF$_3$)$_2$, LiOC H$_2$CH(Me)OC($^n$Pr)(CF$_3$)$_2$, LiOCH$_2$CH(Me)OC($^i$P r)(CF$_3$)$_2$, LiOCH$_2$CH(Me)OC($^n$Bu)(CF$_3$)$_2$, LiOCH $_2$CH(Me)OC($^t$Bu)(CF$_3$)$_2$,

LiOCH$_2$CH(Me)OC($^s$Bu)(CF$_3$)$_2$, LiOCH$_2$CH(M e) OC($^i$Bu)(CF$_3$)$_2$, LiO(CH$_2$)$_3$OCH$_2$CF$_3$, LiO(CH$_2$) $_3$OCH(CF$_3$)$_2$, LiO(CH$_2$)$_3$OC(CF$_3$)$_3$, LiO(CH$_2$)$_3$OC (CH$_3$)(CF$_3$)$_2$, LiO(CH$_2$)$_3$OC(CH$_3$)$_2$(CF$_3$), LiO(CH $_2$)$_3$OC(Et)$_2$(CF$_3$), LiO(CH$_2$)$_3$OC(Pr)$_2$(CF$_3$), LiO (CH$_2$)$_3$OC($^n$Bu)$_2$(CF$_3$), LiO(CH$_2$)$_3$OC($^i$Bu)$_2$(CF $_3$), LiO(CH$_2$)$_3$OC($^t$Bu)$_2$(CF$_3$), LiO(CH$_2$)$_3$OC($^s$B u) $_2$(CF$_3$), LiO(CH$_2$)$_3$OC(MeEt)(CF$_3$), LiO(CH$_2$) $_3$OC(MePr)(CF$_3$), LiO(CH$_2$)$_3$OC(Me$^n$Bu)(CF$_3$), L iO(CH$_2$)$_3$OC(Me$^i$Bu)(CF$_3$), LiO(CH$_2$)$_3$OC(Me$^t$B u) (CF$_3$), LiO(CH$_2$)$_3$OC(Me$^s$Bu)(CF$_3$), LiO(CH$_2$) $_3$OCH$_2$(CF$_2$CF$_3$), LiO(CH$_2$)$_3$OCH(CF$_2$CF$_3$)$_2$, LiO(C H$_2$)$_3$OC(CH$_3$)$_2$(CF$_2$CF$_3$), LiO(CH$_2$)$_3$OC(MeEt) (CF $_2$CF$_3$), LiO(CH$_2$)$_3$OC(MePr) (CF$_2$CF$_3$), LiO(CH$_2$)$_3$ OC(MeBu)(CF$_2$CF$_3$), LiO(CH$_2$)$_3$OC(Me$^n$Bu)(CF$_2$ CF$_3$), LiO(CH$_2$)$_3$OC(Me$^t$Bu)(CF$_2$CF$_3$), LiO(CH$_2$)$_3$ OC(Me$^s$Bu)(CF$_2$CF$_3$), LiO(CH$_2$)$_3$OC(CH$_3$)(CF$_2$CF $_3$)$_2$, LiO(CH$_2$)$_3$OC(CF$_2$CF$_3$)$_3$, LiO(CH$_2$)$_3$OC(CH$_2$C H=CH$_2$)(CF$_3$)$_2$, LiO(CH$_2$)$_3$OC(Et) (CF$_3$)$_2$, LiO(C H$_2$)$_3$OC($^n$Pr)(CF$_3$)$_2$, LiO(CH$_2$)$_3$OC($^i$Pr)(CF$_3$)$_2$, LiO(CH$_2$)$_3$OC($^n$Bu)(CF$_3$)$_2$, LiO(CH$_2$)$_3$OC($^t$Bu)(C F$_3$)$_2$, LiO(CH$_2$)$_3$OC($^s$Bu)(CF$_3$)$_2$, LiO(CH$_2$)$_3$OC($^i$ Bu) (CF$_3$)$_2$, LiO(CH$_2$)$_2$CH(Me)OCH$_2$CF$_3$, LiO(CH $_2$)$_2$CH(Me)OCH(CF$_3$)$_2$, LiO(CH$_2$)$_2$CH(Me)OC(CF $_3$)$_3$, LiO(CH$_2$)$_2$CH(Me)OC(CH$_3$)(CF$_3$)$_2$, LiO(CH$_2$) $_2$CH(Me) OC(CH$_3$)$_2$(CF$_3$), LiO(CH$_2$)$_2$CH(Me)OC(E t)$_2$(CF$_3$), LiO(CH$_2$)$_2$CH(Me)OC(Pr)$_2$(CF$_3$), LiO (CH$_2$)$_2$CH(Me) OC ($^n$Bu)$_2$ (CF$_3$), L iO(CH$_2$)$_2$CH(M e) OC($^i$Bu)$_2$(CF$_3$),

L iO(CH$_2$)$_2$CH(Me)OC($^t$Bu)$_2$(CF$_3$), L iO(CH$_2$) $_2$CH(Me)OC($^s$Bu)$_2$(CF$_3$), LiO(CH$_2$)2CH(Me)OC (MeEt)(CF$_3$), LiO(CH$_2$)$_2$CH(Me)OC(MePr)(CF $_3$), LiO(CH$_2$)$_2$CH(Me)OC(Me$^n$Bu)(CF$_3$), LiO(CH $_2$)$_2$CH(Me)OC(Me$^i$Bu)(CF$_3$), LiO(CH$_2$)$_2$CH(Me)O C(Me$^t$Bu)(CF$_3$), LiO(CH$_2$)$_2$CH(Me)OC(Me$^s$Bu) (CF$_3$), LiO(CH$_2$)$_2$CH(Me)OCH$_2$(CF$_2$CF$_3$), LiO(CH $_2$)$_2$CH(Me)**OCH**(CF$_2$CF$_3$)$_2$, LiO(CH$_2$)$_2$CH(Me)**OC** (CH$_3$)$_2$(CF$_2$CF$_3$), L i O(CH$_2$)$_2$CH(Me) **OC** (MeEt) (C F$_2$CF$_3$), L i O (CH$_2$)$_2$CH(Me) **OC** (MePr)(CF$_2$CF$_3$), L iO(CH$_2$)$_2$CH(Me) **OC** (Me$^i$Bu) (CF$_2$CF$_3$), L i O (CH$_2$) $_2$CH(Me) **OC**(Me$^n$Bu) (CF$_2$CF$_3$), L i O (CH$_2$)$_2$CH(Me) **OC** (Me$^t$Bu)(CF$_2$CF$_3$), L i O (CH$_2$)$_2$CH(Me) **OC** (Me$^s$B u)(CF$_2$CF$_3$), L i O(CH$_2$)**2CH** (Me) **OC**(CH$_3$)(CF$_2$CF $_3$)$_2$, L i O(CH$_2$)$_2$CH(Me) **OC**(CF$_2$CF$_3$)$_3$, L i O (CH$_2$)$_2$C H (Me) **OC**(CH$_2$CH=CH$_2$)(CF$_3$)$_2$, L i O (CH$_2$)$_2$CH(Me) **OC** (Et)(CF$_3$)$_2$, L i O (CH$_2$)$_2$CH(Me) **OC** ($^n$Pr) (CF$_3$) $_2$, L i O (CH$_2$)$_2$CH(Me) OC (iPr)(CF$_3$)$_2$, L i O (CH$_2$)2 CH (Me)OC($^n$Bu)(CF$_3$)$_2$, L i O (CH$_2$)$_2$CH(Me)OC($^t$B u) (CF$_3$)$_2$, L i O (CH$_2$)$_2$CH(Me)OC($^s$Bu)(CF$_3$)$_2$, L i O (CH$_2$)$_2$CH(Me) OC($^i$Bu)(CF$_3$)$_2$, L i OCH (Me) (CH $_2$)$_2$OCH$_2$CF$_3$, L i OCH (Me) (CH$_2$)$_2$OCH(CF$_3$)$_2$, L i OCH (Me)(CH$_2$)$_2$OC(CF$_3$)$_3$, LiOCH(Me) (CH$_2$)$_2$OC(CH $_3$)(CF$_3$)$_2$, LiOCH(Me) (CH$_2$)$_2$OC(CH$_3$)$_2$(CF$_3$), L i O CH (Me) (CH$_2$)$_2$OC(Et)$_2$(CF$_3$), L i OCH (Me) (CH$_2$)$_2$ OC (Pr)$_2$(CF$_3$), L i OCH (Me) (CH$_2$)$_2$OC($^n$Bu)$_2$(CF $_3$), L i OCH (Me) (CH$_2$)$_2$OC($^i$Bu)$_2$(CF$_3$), L i OCH (M e) (CH$_2$) 2OC($^t$Bu)$_2$(CF$_3$), L i OCH (Me) (CH$_2$)$_2$OC ($^s$Bu)$_2$(CF$_3$), LiOCH(Me) (CH$_2$)$_2$OC(MeEt) (CF $_3$), LiOCH(Me) (CH$_2$)$_2$OC(MePr) (CF$_3$), LiOCH(M e) (CH$_2$)$_2$OC(Me$^n$Bu)(CF$_3$), L i OCH (Me)(CH$_2$)$_2$OC (Me Bu) (CF$_3$),

LiOCH(Me)(CH$_2$)$_2$OC(Me$^t$Bu)(CF$_3$), L i OCH (Me) (CH$_2$)$_2$OC(Me$^s$Bu)(CF$_3$), L i OCH (Me) (CH$_2$)$_2$ OCH$_2$ (CF$_2$CF$_3$), L i OCH (Me) (CH$_2$)$_2$OCH(CF$_2$CF$_3$)$_2$, L i OCH (Me) (CH$_2$)$_2$OC(CH$_3$)$_2$(CF$_2$CF$_3$), L i OCH (M e) (CH$_2$)$_2$OC(MeEt) (CF$_2$CF$_3$), LiOCH(Me) (CH$_2$)$_2$ OC (MePr)(CF$_2$CF$_3$), LiOCH(Me) (CH$_2$)$_2$OC(Me$^i$B u) (CF$_2$CF$_3$), LiOCH(Me) (CH$_2$)$_2$OC(MenBu) (CF$_2$ CF$_3$), LiOCH(Me) (CH$_2$)$_2$OC(Me$^t$Bu)(CF$_2$CF$_3$), L i OCH (Me) (CH$_2$)$_2$OC(Me$^s$Bu)(CF$_2$CF$_3$), L iOCH(M e) (CH$_2$)$_2$OC(CH$_3$)(CF$_2$CF$_3$)$_2$, LiOCH(Me) (CH$_2$)$_2$O C (CF$_2$CF$_3$)3, LiOCH(Me) (CH$_2$)$_2$OC(CH$_2$CH=CH$_2$) (CF$_3$)$_2$, LiOCH(Me) (CH$_2$)$_2$OC(Et)(CF$_3$)$_2$, LiOCH (Me) (CH$_2$)$_2$OC($^n$Pr)(CF$_3$)$_2$, L i OCH (Me) (CH$_2$)$_2$O C($^i$Pr)(CF$_3$)$_2$, L i OCH (Me) (CH$_2$)$_2$OC($^n$Bu)(CF$_3$) $_2$, LiOCH(Me) (CH$_2$)$_2$OC($^t$Bu)(CF$_3$)$_2$, LiOCH(Me) (CH$_2$)$_2$OC($^s$Bu)(CF$_3$)$_2$, LiOCH(Me) (CH$_2$)2OC($^i$B u) (CF$_3$)$_2$, LiOC(Me)$_2$(CH$_2$)$_2$OCH$_2$CF$_3$, LiOC(Me)$_2$ (CH$_2$)2OCH(CF$_3$)$_2$, LiOC(Me) 2 (CH$_2$)2OC(CF$_3$)$_3$, LiOC(Me)$_2$ (CH$_2$)$_2$OC(CH$_3$)(CF$_3$)$_2$, LiOC(Me)$_2$(C H$_2$)$_2$OC(CH$_3$)$_2$(CF$_3$), LiOC(Me)$_2$(CH$_2$)$_2$OC(Et)$_2$ (CF$_3$), LiOC(Me)$_2$ (CH$_2$)$_2$OC(Pr)$_2$(CF$_3$), LiOC(M e) $_2$(CH$_2$)$_2$OC(nBu) $_2$(CF$_3$), L iOC(Me) $_2$(CH$_2$)$_2$OC ($^i$Bu)$_2$(CF$_3$), LiOC(Me)$_2$(CH$_2$)$_2$OC($^{t-}$Bu)$_2$(CF$_3$), L iOC(Me) $_2$(CH$_2$)$_2$OC($^s$Bu)$_2$(CF$_3$),

L i OC(Me)$_2$(CH$_2$)$_2$OC(MeEt)(CF$_3$), L iOC(M e) $_2$(CH$_2$)$_2$OC(MePr)(CF$_3$), LiOC(Me)$_2$(CH$_2$)$_2$OC (Me$^n$Bu)(CF$_3$), L iOC(Me) $_2$(CH$_2$)$_2$OC(Me i Bu) (C F$_3$), LiOC(Me)$_2$(CH$_2$)$_2$OC(Me$^t$Bu)(CF$_3$), LiOC(M e) $_2$(CH$_2$)$_2$OC(Me$^s$Bu)(CF$_3$), L i OC(Me) $_2$(CH$_2$)$_2$OC H$_2$ (CF$_2$CF$_3$), L iOC(Me) $_2$(CH$_2$)$_2$OCH(CF$_2$CF$_3$)$_2$, L i OC (Me) $_2$(CH$_2$)$_2$OC(CH3) $_2$(CF$_2$CF$_3$), L iOC(Me)$_2$ (CH$_2$)$_2$OC(MeEt) (CF$_2$CF$_3$), LiOC(Me) $_2$(CH$_2$)$_2$OC (MePr) (CF$_2$CF$_3$), LiOC(Me) $_2$(CH$_2$)$_2$OC(Me Bu) (CF$_2$CF$_3$), LiOC(Me) $_2$(CH$_2$)$_2$OC(Me$^n$Bu)(CF$_2$CF $_3$), LiOC(Me) $_2$(CH$_2$)$_2$OC(Me$^t$Bu)(CF$_2$CF$_3$), L iOC (Me) $_2$(CH$_2$)$_2$OC(Me$^s$Bu)(CF$_2$CF$_3$), L iOC(Me) $_2$(C H$_2$)$_2$OC(CH$_3$)(CF$_2$CF$_3$)2, L iOC(Me) $_2$(CH$_2$)$_2$OC(CF $_2$CF$_3$)$_3$, L iOC(Me) $_2$(CH$_2$)$_2$OC(CH$_2$CH=CH$_2$)(CF$_3$) $_2$, L iOC(Me) $_2$(CH$_2$)$_2$OC(Et) (CF$_3$)$_2$, L iOC(Me)$_2$ (CH$_2$)$_2$OC($^n$Pr)(CF$_3$)$_2$, LiOC(Me) $_2$(CH$_2$)$_2$OC($^i$P r) (CF$_3$)$_2$LiOC(Me)$_2$

(CH$_2$)$_2$OC($^n$Bu)(CF$_3$)$_2$, LiOC (Me)$_2$(CH$_2$)$_2$OC($^t$Bu)(CF$_3$)$_2$, LiOC(Me)$_2$(CH$_2$)$_2$O C ($^s$Bu)(CF$_3$)$_2$, LiOC(Me)$_2$(CH$_2$)$_2$OC($^i$Bu) (CF$_3$) $_2$, L iOC(Me) $_2$(CH$_2$)$_2$OCH$_2$CF$_3$, L iOC(Me) $_2$(CH$_2$)$_2$O CH (CF$_3$)$_2$, LiOC(Me) $_2$(CH$_2$)$_2$OC(CF$_3$)$_3$, LiOC(M e) $_2$ (CH$_2$)$_2$OC (CH$_3$)(CF$_3$)$_2$, L iOC(Me) $_2$(CH$_2$)$_2$OC (CH$_3$)$_2$(CF$_3$), L iOC(Me) $_2$(CH$_2$)$_2$OC(CH$_2$CH=CH$_2$) (CF$_3$)$_2$, L iOC(Me) $_2$(CH$_2$)$_2$OC(Et)(CF$_3$)$_2$, L iOC (Me) $_2$(CH$_2$)$_2$OC($^n$Pr)(CF$_3$)$_2$, LiOC(Me)$_2$(CH$_2$)$_2$O C ($^i$Pr)(CF$_3$)$_2$, LiOC(Me)$_2$(CH$_2$)$_2$OC($^n$Bu) (CF$_3$) $_2$, LiOC(Me)$_2$(CH$_2$)$_2$OC($^t$Bu)(CF$_3$)$_2$, LiOC(Me)$_2$ (CH$_2$)$_2$OC(sBu) (CF$_3$)$_2$, LiOC(Me) $_2$(CH$_2$)$_2$OC($^i$B u) (CF$_3$)$_2$, LiOC(CH$_2$)$_2$(Me) $_2$OCH$_2$CF$_3$, L iOC(CH$_2$) $_2$(Me) $_2$OCH (CF$_3$)$_2$, L iOC(CH$_2$)$_2$(Me) $_2$OC(CF$_3$)$_3$, L iOC(CH$_2$)$_2$(Me) $_2$OC(CH$_3$)(CF$_3$)$_2$, L iOC(CH$_2$)$_2$(M e) $_2$OC(CH$_3$)$_2$(CF$_3$), L iOC(CH$_2$)$_2$(Me) $_2$OC(Et)$_2$(C F$_3$) , LiOC(CH$_2$)$_2$(Me) $_2$OC(Pr)$_2$ (CF$_3$), LiOC(CH $_2$)$_2$(Me) $_2$OC($^n$Bu)$_2$(CF$_3$), LiOC(CH$_2$)$_2$(Me)$_2$OC($^i$ Bu)$_2$(CF$_3$), L iOC(CH$_2$)$_2$(Me) $_2$OC($^t$Bu)$_2$(CF$_3$), L i OC (CH$_2$)$_2$(Me) $_2$OC($^s$Bu)$_2$(CF$_3$), L iOC(CH$_2$)$_2$(Me) $_2$OC(MeEt) (CF$_3$), L iOC(CH$_2$)$_2$(Me) $_2$OC(MePr) (CF$_3$), LiOC(CH$_2$)$_2$(Me) $_2$OC(Me$^n$Bu)(CF$_3$), LiOC (CH$_2$)$_2$(Me) $_2$OC(Me Bu) (CF$_3$),

L iOC(CH$_2$)$_2$(Me)$_2$OC(Me$^t$Bu)(CF$_3$), LiOC(C H$_2$)$_2$(Me) $_2$OC(Me$^s$Bu)(CF$_3$), L iOC(CH$_2$)$_2$(Me) $_2$OC H$_2$ (CF$_2$CF$_3$), LiOC(CH$_2$)$_2$(Me) $_2$OCH(CF$_2$CF$_3$)$_2$, L i OC (CH$_2$)$_2$(Me) $_2$OC(CH$_3$)$_2$(CF$_2$CF$_3$), LiOC(CH$_2$)$_2$ (Me) $_2$OC(MeEt) (CF$_2$CF$_3$), LiOC(CH$_2$)$_2$(Me) $_2$OC (MePr)(CF$_2$CF$_3$), LiOC(CH$_2$)$_2$(Me) $_2$OC(Me Bu) (CF$_2$CF$_3$), LiOC(CH$_2$)$_2$(Me) $_2$OC(Me$^n$Bu)(CF$_2$CF $_3$), LiOC(CH$_2$)$_2$(Me) $_2$OC(MetBu)(CF$_2$CF$_3$), LiOC (CH$_2$)$_2$(Me) $_2$OC(Me$^s$Bu)(CF$_2$CF$_3$), LiOC(CH$_2$)$_2$(M e) $_2$OC(CH$_3$) (CF$_2$CF$_3$)$_2$LiOC(CH$_2$)$_2$(Me) $_2$OC(CF$_2$C F$_3$)$_3$, LiOC(CH$_2$)$_2$(Me) $_2$OC(CH$_2$CH=CH$_2$)(CF$_3$)$_2$, L i OC (CH$_2$)$_2$(Me) $_2$OC(Et) (CF$_3$)$_2$, LiOC(CH$_2$)$_2$(M e) $_2$OC($^n$Pr)(CF$_3$)$_2$, LiOC(CH$_2$)$_2$(Me) $_2$OC($^i$Pr)(C F$_3$)$_2$, LiOC(CH$_2$)$_2$(Me) $_2$OC($^n$Bu)(CF$_3$)$_2$, LiOC(CH $_2$)$_2$(Me) $_2$OC($^t$Bu)(CF$_3$)$_2$, LiOC(CH$_2$)$_2$(Me)$_2$OC($^s$ Bu) (CF$_3$)$_2$, LiOC(CH$_2$)$_2$(Me)$_2$OC($^i$Bu)(CF$_3$)$_2$,

L i OCH [(CH$_2$)$_2$OCH$_2$CF$_3$]$_2$, LiOCH[(CH$_2$)$_2$OC H (CF$_3$)$_2$]$_2$, LiOCH[(CH$_2$)$_2$OC(CF$_3$)$_3$]$_2$, LiOCH[(C H$_2$)$_2$OC(CH$_3$) (CF$_3$)$_2$]$_2$, **L i OCH** [(CH$_2$)$_2$OC(CH$_3$)$_2$ (CF$_3$)] $_2$, LiOCH[(CH$_2$)$_2$OC(Et)$_2$(CF$_3$)]$_2$, L i OCH [(CH$_2$)$_2$OC(Pr)$_2$(CF$_3$)]$_2$, LiOCH [(CH$_2$)$_2$OC($^n$Bu) $_2$(CF$_3$)] $_2$, LiOCH[(CH$_2$)$_2$OC($^i$Bu)$_2$(CF$_3$)] $_2$, L i OC H [(CH$_2$)$_2$OC($^t$Bu)$_2$(CF$_3$)] $_2$, LiOCH[(CH$_2$)$_2$OC($^s$B u) $_2$ (CF$_3$)]$_2$, LiOCH[(CH$_2$)$_2$OC(MeEt) (CF$_3$)]$_2$, L i OCH [(CH$_2$)$_2$OC(MePr)(CF$_3$)]2, L i OCH [(CH$_2$)$_2$ OC (Me$^n$Bu)(CF$_3$)] $_2$, LiOCH [(CH$_2$)$_2$OC(Me$^i$Bu)(C F$_3$)] $_2$, L i OCH [(CH$_2$)$_2$OC(Me$^t$Bu)(CF$_3$)] $_2$, LiOCH [(CH$_2$)$_2$OC(Me$^s$Bu)(CF$_3$)] $_2$, L i OCH [(CH$_2$)$_2$OCH$_2$ (CF$_2$CF$_3$)] $_2$, LiOCH[(CH$_2$)$_2$OCH(CF$_2$CF$_3$)$_2$]$_2$, L iO CH [(CH$_2$)$_2$OC(CH$_3$)$_2$(CF$_2$CF$_3$)] $_2$LiOCH[(CH$_2$)$_2$OC (MeEt)(CF$_2$CF$_3$)] $_2$, LiOCH[(CH$_2$)$_2$OC(MePr)(C F$_2$CF$_3$)] $_2$, LiOCH[(CH$_2$)$_2$OC(Me Bu) (CF$_2$CF$_3$)]$_2$, L i OCH [(CH$_2$)$_2$OC(Me$^n$Bu)(CF$_2$CF$_3$)]$_2$, L i OCH [(C H$_2$)$_2$OC(Me$^t$Bu)(CF$_2$CF$_3$)]$_2$, L i OCH [(CH$_2$)$_2$OC(Me $^s$Bu)(CF$_2$CF$_3$)]$_2$, L i OCH [(CH$_2$)$_2$OC(CH$_3$)(CF$_2$CF $_3$)$_2$]$_2$, LiOCH[(CH$_2$)$_2$OC(CF$_2$CF$_3$)3] $_2$, L i OCH [(CH $_2$)$_2$OC(CH$_2$CH=CH$_2$)(CF$_3$)$_2$]$_2$, L i OCH [(CH$_2$)$_2$OC(E t) (CF$_3$)$_2$]$_2$, LiOCH[(CH$_2$)$_2$OC($^n$Pr)(CF$_3$)$_2$]$_2$, L i OCH [(CH$_2$)$_2$OC($^i$Pr)(CF$_3$)$_2$]$_2$, L i OCH [(CH$_2$)$_2$OC ($^n$Bu) (CF$_3$)$_2$]$_2$, L i OCH [(CH$_2$)$_2$OC ($^t$Bu)(CF$_3$)$_2$] $_2$, L i OCH [(CH$_2$)$_2$OC($^s$Bu)(CF$_3$)$_2$]$_2$, L i OCH [(CH$_2$) $_2$OC($^i$Bu)(CF$_3$)$_2$]$_2$, L iOC(Et) $_2$(CF$_2$H), L i OC (Et) $_2$(CFH$_2$) ,L i OC (Et)$_2$(CF$_3$), L i OCH (CF$_2$H)$_2$, L i SCH (CF$_3$)$_2$, LiSeCH(CF$_3$)$_2$, L i TeCH (CF$_3$)$_2$

Method for producing chemical vapor deposition material

Chemical vapor deposition material can usually be produced in accordance with scheme (A) below.

[Chemical notation 4]

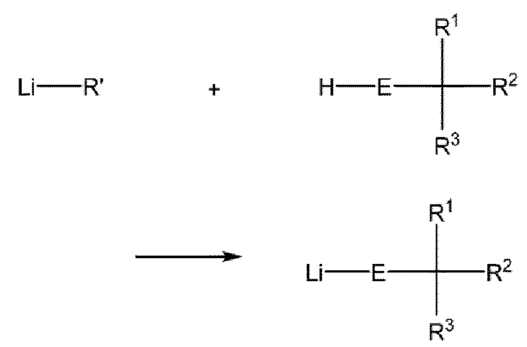

(A)

(R' is a hydrocarbon group. $R^1$, $R^2$ and $R^3$ are as defined for abovementioned formula (1).)

The following procedure is a specific example of the production method. A round-bottomed flask with a dropping funnel attached is prepared. This round-bottomed flask is attached to a distillation unit. An organic solvent having a specified amount of Li source (e.g. n-butyllithium) dissolved therein is introduced into the dropping funnel. Then an organic solvent having a specified amount of carbon source (e.g. HOCH(CF$_3$)$_2$) dissolved therein is introduced into the round-bottomed flask. Next, while cooling the dropping funnel to around -30°C to -10°C, the organic solvent in the dropping funnel is agitated and introduced dropwise into the round-bottomed flask. This affords a precipitate. The organic solvent is removed from the resulting precipitate (e.g. by creating a vacuum in the round-bottomed flask) to obtain a solid. The resulting solid is then subjected to further decrease in pressure (e.g. <0.01 kPa), and the round-bottomed flask is heated to around 100°C to 180°C. This yields the target chemical vapor deposition material. The various chemical vapor deposition

materials of the abovementioned embodiments are obtained by changing the specified carbon source as appropriate.

Film production method

The film production method of the present embodiment includes an introduction step wherein the abovementioned chemical vapor deposition material is introduced into a reactor that houses a substrate; and a formation step wherein at least some of said chemical vapor deposition material is deposited onto said substrate.

Introduction step

In this step, the abovementioned chemical vapor deposition material is introduced into a reactor that houses a substrate. The type of substrate onto which a film is to be deposited is selected as appropriate for the end-use. Examples of substrates are Si substrate and electrodes for batteries.
The reactor may be any closed vessel or chamber of a device inside which vapor deposition can be performed. Specific examples include, but are not limited to, parallel plate type reactors, cold wall type reactors, hot wall type reactors, single-wafer reactors, multi-wafer reactors, and other types of deposition system.
Next, gas containing vaporized chemical vapor deposition material is introduced into the reactor. Pure (single) compound or blended (multiple) compounds in the liquid state may be supplied into a vaporizer and vaporized before being introduced into the reactor. Or the chemical vapor deposition material can be vaporized by passing carrier gas through a vessel containing the chemical vapor deposition material, or by bubbling carrier gas through the chemical vapor deposition material. Next, gas containing the carrier gas and vaporized compound(s) is introduced into the reactor. If necessary, the vessel may be heated to a temperature at which the chemical vapor deposition material will have sufficient vapor pressure. There are no limitations on the carrier gas; Ar, He, $N_2$, and mixtures of these can be used. Or, instead of adopting the method that involves bubbling carrier gas (known as the bubbling method), the compound(s) can be vaporized by direct liquid injection (DLI).
In the introduction step, coreactant can also be introduced into the reactor. The coreactant is preferably chosen from the group consisting of $O_2$, $O_3$, $H_2O$, $H_2O_2$, NO, $N_2O$, $NO_2$, trimethyl phosphate (also referred to as TMPO), oxygen radicals thereof, and mixtures thereof. Other coreactants, such as alcohols, ammonia, polyamines, hydrazine, dimethylethyl phosphoramidates, and sulfates, can also be used.
The vessel is maintained at a temperature in the range of 0°C to about 150°C, for example. Those skilled in the art will understand that the amount of vaporized compound can be controlled by regulating the vessel temperature by a known method.
The reactor is maintained at a pressure in the range of about 0.5 mTorr to about 20 Torr.

Formation step

In the formation step, the abovementioned chemical vapor deposition material is vapor-deposited onto the substrate to form a film.
In this step, in one example of an atomic layer deposition type process, chemical vapor deposition material in the gas phase is introduced into the reactor where it comes into contact with a suitable substrate. Then excess chemical vapor deposition material can be removed from the reactor by purging and/or venting the reactor. Coreactant is introduced into the reactor and reacts in a self-terminating manner with the absorbed chemical vapor deposition material. Excess coreactant is removed from the reactor by purging and/or venting the reactor. When the desired film is a metal oxide film, this 2-stage process may provide the desired film thickness, or this 2-stage process may be repeated until film of the required thickness is obtained.
Or, when the desired film is a metal oxide film, after the abovementioned 2-stage process, the introduction of chemical vapor deposition material vapor into the reactor can be continued. This chemical vapor deposition material is chosen based on the properties of the metal oxide to be deposited. After its introduction into the reactor, the compound comes into contact with the substrate. Excess compound is removed from the reactor by purging and/or venting the reactor. Coreactant may again be introduced into the reactor to react with the chemical vapor deposition material; this coreactant can be the same as or different from the coreactant in the abovementioned 2-stage process. It is preferable to introduce into the reactor a coreactant that is different from the coreactant in the abovementioned 2-stage process. Excess coreactant is removed from the reactor by purging and/or venting the reactor. If the desired film thickness is achieved, this process may be ended. However, if a thicker film is desired, the entire 4-stage process can be repeated. The number of repeat cycles of the 4-stage process is preferably 50-1000 cycles, more preferably 100-800 cycles, and even more preferably 200-600 cycles. Films of the desired composition and thickness can be deposited by alternating the supply of chemical vapor deposition material and coreactant. It is also possible to repeat a 6-stage process using multiple types (e.g. 2 types) of coreactant respectively. The number of repeat cycles of the 6-stage process is, for example, 100 cycles to 600

cycles.

This step may be performed in the presence of at least one coreactant chosen from the group consisting of $O_2$, $O_3$, $H_2O$, $H_2O_2$, NO, $NO_2$, $N_2O$, $PH_3$ and TMPO. Specifically, adding coreactant into the reactor allows a film to form on the substrate. In this step the temperature of the reactor is preferably ≥130°C to ≤350°C, more preferably ≥130°C to ≤300°C, even more preferably ≥130°C to ≤250°C, and particularly preferably ≥130°C to ≤200°C. By using a relatively low reactor temperature it is possible to form films on heat-sensitive substrates. For example, it is possible to form films on lithium ion battery electrodes.

In another embodiment, the temperature of the reactor in this step is preferably ≥130°C to ≤350°C, more preferably ≥150°C to ≤350°C, even more preferably ≥170°C to ≤350°C, and particularly preferably ≥200°C to ≤350°C. By using a relatively high reactor temperature it is possible to form films at high growth rates.

In this embodiment the film growth rate per cycle is preferably ≥0.05 Å to ≤0.7 Å, more preferably ≥0.08 Å to ≤0.6 Å, and even more preferably ≥0.1 Å to ≤0.5 Å. The faster the film growth rate, the more efficiently film can be formed.

Compounds contained in the film

Film produced by the film production method described above contains metal oxyfluoride. Here, the metal oxyfluoride contains Li, oxygen and fluorine.

Film produced by the film production method described above contains a compound represented by $Li_xO_yF_z$. Here, the compound is such that $2.6 \leq x \leq 4.3$, $1.8 \leq y \leq 2.3$, and $4.3 \leq z \leq 6.6$. The composition of the compound can be calculated from analysis results obtained using a commercially available elemental analyzer, and stoichiometric calculation using those analysis results. It should be noted that the compound contained in the film may be substantially $Li_xO_yF_z$ (≥99% by mass $Li_xO_yF_z$) or may contain impurities (e.g. LiOF and/or LiCF).

The film produced by the film production method described above has a reflection coefficient of ≥1.1 to ≤1.5. The reflection coefficient can be measured using a commercially available spectroscopic ellipsometer (i.e., an ellipsometer).

Film

The film in the present embodiment contains a compound represented by $Li_xO_yF_z$, wherein $2.6 \leq x \leq 4.3$, $1.8 \leq y \leq 2.3$, and $4.3 \leq z \leq 6.6$. The composition of the compound can be calculated from analysis results obtained using a commercially available elemental analyzer, and stoichiometric calculation using those analysis results. Here, the compound $Li_xO_yF_z$ contained in the film constitutes preferably ≥50% by mass, more preferably ≥60% by mass, even more preferably ≥70% by mass, and particularly preferably ≥90% by mass, thereof.

The film of the present embodiment has a reflection coefficient of ≥1.1 to ≤1.5. The reflection coefficient can be measured using a commercially available spectroscopic ellipsometer. Because it has a relatively low reflection coefficient, the film will have multiple uses.

The film thickness is preferably ≥0.1 nm to ≤100 nm, more preferably ≥1 nm to ≤50 nm.

Uses

The chemical vapor deposition material and film of the present embodiment are useful in the production of lithium oxide, lithium oxyfluoride or alkali-containing mixed metal oxyfluoride films, for various uses such as coating lithium metal, and coating cathodes and anodes in lithium batteries. Lithium oxyfluoride films have improved resistance to oxidizing agents; this improved resistance can bestow the solid electrolyte mesophase with improved resistance to the gradual deterioration that decreases battery-charging efficiency.

Examples

The examples below are provided to illustrate use the disclosure of the present specification, and it should be fully understood that any specific embodiment or group of embodiments of the present invention may not necessarily afford all of the advantages of the process described in the present specification. Although specific embodiments and examples are disclosed below, it will be understood by those skilled in the art that the present invention includes obvious modifications and exceeds the specifically disclosed inventive embodiments and/or uses. Therefore, it should be understood that the scope of the disclosed present invention is not limited to the specific embodiments described below.

Synthesis of chemical vapor deposition material

A 500 mL round-bottomed flask with an attached 100 mL dropping funnel was prepared. A distillation unit was attached to this round-bottomed flask. A 250 mL round-bottomed flask was attached to the distillation unit. Then 58 mL n-hexane (n-hexane manufactured by Kanto Chemical Co., Inc.) having 2.6 M n-butyllithium (manufactured by Kanto Chemical Co., Inc.) dissolved therein was introduced into the dropping funnel. Then 200 mL n-hexane having 150 mmol $HOCH(CF_3)_2$ (manufactured by Tokyo Chemical Industry Co., Ltd.) dissolved therein was introduced into the 500 mL round-bottomed flask. Next, while cooling the dropping funnel to -20°C, the n-hexane in the dropping funnel was agitated and introduced dropwise into the 500 mL round-bottomed flask. This afforded a precipitate. The n-hexane was removed from the resulting precipitate, to obtain a solid. The resulting solid was then subjected to further decrease in pressure to vacuum (<0.01 kPa), and the 500 mL round-bottomed flask was heated to around 150°C. This yielded the target chemical vapor deposition material.

The 1H NMR (C6D6, 400 MHz) results were as follows. From these results it was confirmed that the target chemical vapor deposition material was $Li\text{-}O\text{-}CH(CF_3)_2$. A Bruker Ascend 400 MHz NMR unit was used for the NMR measurements.

1H NMR (C6D6, 400MHz) : δ 3. 988 (1H, d, O-CH $(CF_3)_2$) ; 1 3C NMR (C6D6/D6-DMSO 101MHz) : 6 8. 827 (m, O-CH $(CF_3)_2$), 123. 1 (q, 1JCF=288. 6 Hz, O-CH $(CF_3)_2$) ; 19F NMR (C6D6, 376MHz) : -7 8. 415 (s, $Li\text{-}O\text{-}CH(CF_3)_2$) ; 1 L i NMR (C6D6, 15 6MHz) : 0. 211 (s, $Li\text{-}O\text{-}CH(CF_3)_2$) .

## Film formation

### Sample 1

The $Li\text{-}O\text{-}CH(CF_3)_2$ synthesized in "Synthesis of chemical vapor deposition material" described above was introduced into a vessel (purchased from Japan Advanced Chemicals Co., Ltd). Then the inside of the vessel was set at 78°C. At this point, the pressure was 10 Torr. The vessel was connected to allow the gas in the vessel to be supplied to the reactor.

Next, Si substrate (produced by Mitsubishi Materials Corporation, product name: P-type Silicon Wafer) that had been washed using 1 vol% HF solution was introduced into the reactor. The temperature and pressure inside the reactor were set at 200°C and 1 Torr, the atmosphere surrounding the substrate was changed as described below, and 400 cycles were repeated. This resulted in the formation of a film.

### Changes of atmosphere

4 sccm $Li\text{-}O\text{-}CH(CF_3)_2$ (2.5 s)
300 sccm nitrogen gas (30 s)
20 sccm $H_2O$ gas (1 s)
300 sccm nitrogen gas (30 s)
This series (1) to (4) constituted 1 cycle.

### Sample 2

A film was formed by the same method as for Sample 1, except that in "Changes of atmosphere", step (1) was 5 s.

### Sample 3

A film was formed by the same method as for Sample 1, except that in "Changes of atmosphere", step (1) was 10 s.

### Sample 4

A film was formed by the same method as for Sample 1, except that in "Changes of atmosphere", step (1) was 15 s.

### Sample 5

A film was formed by the same method as for Sample 1, except that the temperature inside the reactor was 175°C.

Sample 6

A film was formed by the same method as for Sample 1, except for the following.

- The temperature inside the reactor was set at 175°C
- In "Changes of atmosphere", (1) was 5 s

Sample 7

A film was formed by the same method as for Sample 1, except for the following.

- The temperature inside the reactor was set at 175°C
- In "Changes of atmosphere", (1) was 10 s

Sample 8

A film was formed by the same method as for Sample 1, except for the following.

- The temperature inside the reactor was set at 175°C
- In "Changes of atmosphere", (1) was 15 s

Sample 9

A film was formed by the same method as for Sample 1, except for the following.

- The temperature inside the reactor was set at 150°C
- In "Changes of atmosphere", (1) was 15 s
- The number of cycles was 600

Sample 10

The $Li-O-CH(CF_3)_2$ synthesized in "Synthesis of chemical vapor deposition material" described above was introduced into a first vessel (purchased from Japan Advanced Chemicals Co., Ltd). Then the inside of the first vessel was set at 78°C. At this point, the pressure was 20 Torr. TMPO (manufactured by Aldrich) was added into a second vessel (purchased from Japan Advanced Chemicals Co., Ltd). Then the inside of the second vessel was set at 80°C. At this point, the pressure was 20 Torr. The first vessel and the second vessel were connected to allow gas to be supplied to the reactor.
Next, Si substrate (produced by Mitsubishi Materials Corporation, product name: P-type Silicon Wafer) that had been washed using 1 vol% HF solution was introduced into the reactor. The temperature and pressure inside the reactor were set at 200°C and 1 Torr, the atmosphere surrounding the substrate was changed as described below, and 400 cycles were repeated. This resulted in the formation of a film.

Changes of atmosphere

(1) 1 sccm $Li-O-CH(CF_3)_2$ (5 s)
(2) 300 sccm nitrogen gas (5 s)
(3) 30 sccm TMPO (5 s)
(4) 300 sccm nitrogen gas (5 s)
(5) 50 sccm ozone gas (5 s)
(6) 300 sccm nitrogen gas (5 s)

This series (1) to (6) constituted 1 cycle.

Sample 11

A film was formed by the same method as for Sample 10, except that in "Changes of atmosphere", step (1) was 20 s.

Sample 12

A film was formed by the same method as for Sample 10, except that the temperature inside the reactor was 250°C.

Sample 13

A film was formed by the same method as for Sample 10, except for the following.

- The temperature inside the reactor was set at 250°C
- In "Changes of atmosphere", step (1) was 10 s

Sample 14

A film was formed by the same method as for Sample 10, except for the following.

- The temperature inside the reactor was set at 250°C
- In "Changes of atmosphere", step (1) was 15 s

Sample 15

A film was formed by the same method as for Sample 10, except for the following.

- The temperature inside the reactor was set at 250°C
- In "Changes of atmosphere", step (1) was 20 s

Sample 16

A film was formed by the same method as for Sample 10, except for the following.

- The temperature inside the reactor was set at 250°C
- In "Changes of atmosphere", step (1) was 25 s

Sample 17

A film was formed by the same method as for Sample 10, except that the temperature inside the reactor was 275°C.

Sample 18

A film was formed by the same method as for Sample 10, except for the following.

- The temperature inside the reactor was set at 275°C
- In "Changes of atmosphere", step (1) was 10 s

Sample 19

A film was formed by the same method as for Sample 10, except for the following.

- The temperature inside the reactor was set at 275°C
- In "Changes of atmosphere", step (1) was 15 s

Sample 20

A film was formed by the same method as for Sample 10, except for the following.

- The temperature inside the reactor was set at 275°C
- In "Changes of atmosphere", step (1) was 20 s

Sample 21

A film was formed by the same method as for Sample 10, except for the following.

- The temperature inside the reactor was set at 275°C
- In "Changes of atmosphere", step (1) was 25 s

Sample 22

A film was formed by the same method as for Sample 10, except that the temperature inside the reactor was 300°C.

Sample 23

A film was formed by the same method as for Sample 10, except for the following.

- The temperature inside the reactor was set at 300°C
- In "Changes of atmosphere", step (1) was 10 s

Sample 24

A film was formed by the same method as for Sample 10, except for the following.

- The temperature inside the reactor was set at 300°C
- In "Changes of atmosphere", step (1) was 15 s

Sample 25

A film was formed by the same method as for Sample 10, except for the following.

- The temperature inside the reactor was set at 300°C
- In "Changes of atmosphere", step (1) was 20 s

Sample 26

The Li-O-CH$(CF_3)_2$ synthesized in "Synthesis of chemical vapor deposition material" described above was introduced into a vessel (purchased from Japan Advanced Chemicals Co., Ltd). Then the inside of the vessel was set at 78°C. At this point, the pressure was 20 Torr. The vessel was connected to allow gas to be supplied to the reactor.
Next, Si substrate (produced by Mitsubishi Materials Corporation, product name: P-type Silicon Wafer) that had been washed using 1 vol% HF solution was introduced into the reactor. The temperature and pressure inside the reactor were set at 200°C and 1 Torr, the atmosphere surrounding the substrate was changed as described below, and 400 cycles were repeated. This resulted in the formation of a film.

Changes of atmosphere

(1) 1 sccm Li-O-CH$(CF_3)_2$ (5 s)
(2) 300 sccm nitrogen gas (5 s)
(3) 50 sccm ozone gas (5 s)
(4) 300 sccm nitrogen gas (5 s)

This series (1) to (4) constituted 1 cycle.

Sample 27

A film was formed by the same method as for Sample 26, except that in "Changes of atmosphere", step (1) was 20 s.

Sample 28

A film was formed by the same method as for Sample 26, except that the temperature inside the reactor was 250°C.

Sample 29

A film was formed by the same method as for Sample 26, except for the following.

- The temperature inside the reactor was set at 250°C
- In "Changes of atmosphere", (1) was 20 s

Sample 30

A film was formed by the same method as for Sample 26, except that the temperature inside the reactor was 275°C.

Sample 31

A film was formed by the same method as for Sample 26, except for the following.

- The temperature inside the reactor was set at 275°C
- In "Changes of atmosphere", step (1) was 20 s

Sample 32

A film was formed by the same method as for Sample 26, except that the temperature inside the reactor was 300°C.

Sample 33

A film was formed by the same method as for Sample 26, except for the following.

- The temperature inside the reactor was set at 300°C
- In "Changes of atmosphere", step (1) was 20 s

Analysis of the film

The films of Samples 1 to 33 were analyzed using an ellipsometer (manufactured by Semilab, product name: SE-2000). The analysis results (film growth rate (Å (Angstrom)/cycle) and reflection coefficient) for each sample are compiled in Table 1 to Table 3. In the tables, "-" denotes that that component was not used. Also, in the tables, Li-HFIP denotes Li-O-CH$(CF_3)_2$.

[Table 1]

| | Sample | 1 | 2 | 3 | 4 | 5 | 6 | 7 | 8 | 9 |
|---|---|---|---|---|---|---|---|---|---|---|
| Test conditions | Reactor temperature (°C) | 200 | 200 | 200 | 200 | 175 | 175 | 175 | 175 | 150 |
| Type of coreactant | | $H_2O$ | | | | | | | | |
| Chemical vapor deposition material-containing gas flow time (s) | Li-HFIP (4sccm) | 2.5 | 5 | 10 | 15 | 2.5 | 5 | 10 | 15 | 15 |
| | Li-HFIP (1sccm) | - | - | - | - | - | - | - | - | - |

(continued)

| | Sample | 1 | 2 | 3 | 4 | 5 | 6 | 7 | 8 | 9 |
|---|---|---|---|---|---|---|---|---|---|---|
| Analysis results | Growth rate (Angstrom/cycle) | 0.28 | 0.3 | 0.31 | 0.32 | 0.159 | 0.178 | 0.174 | 0.177 | 0.118 |
| | Reflection coefficient | 1.41 | 1.42 | 1.42 | 1.42 | 1.4 | 1.42 | 1.42 | 1.42 | 1.43 |

[Table 2]

| | Sample | 10 | 11 | 12 | 13 | 14 | 15 | 16 | 17 | 18 | 19 | 20 | 21 | 22 | 23 | 24 | 25 |
|---|---|---|---|---|---|---|---|---|---|---|---|---|---|---|---|---|---|
| Test condi- tion s | Reactor tempera- ture (°C) | 200 | 200 | 250 | 250 | 250 | 250 | 250 | 275 | 275 | 275 | 275 | 275 | 300 | 300 | 300 | 300 |
| Type of coreactant | | $TMPO + O_3$ | | | | | | | | | | | | | | | |
| Chemical vapor de- positio n material- containin g gas flow time (s) | Li-HFIP (4sccm) | - | - | - | - | - | - | - | - | - | - | - | - | - | - | - | - |
| | Li-HFIP (1sccm) | 5 | 20 | 5 | 10 | 15 | 20 | 25 | 5 | 10 | 15 | 20 | 25 | 5 | 10 | 15 | 20 |
| Analysis results | Growth rate (Angstrom/cycle ) | 0.1 2 | 0.0 9 | 0.2 2 | 0.2 8 | 0.4 6 | 0.4 2 | 0.4 | 0.3 6 | 0.4 6 | 0.4 9 | 0.5 1 | 0.5 1 | 0.6 1 | 0.5 7 | 0.4 6 | 0.4 4 |
| | Reflection coefficient | 1.3 7 | 1.2 8 | 1.3 3 | 1.3 6 | 1.3 3 | 1.3 3 | 1.3 6 | 1.3 3 | 1.3 3 | 1.3 3 | 1.3 6 | 1.3 6 | 1.3 9 | 1.3 9 | 1.3 9 | 1.3 9 |

[Table 3]

| | Sample | 26 | 27 | 28 | 29 | 30 | 31 | 32 | 33 |
|---|---|---|---|---|---|---|---|---|---|
| Test conditions | Reactor temperature (°C) | 200 | 200 | 250 | 250 | 275 | 275 | 300 | 300 |
| Type of coreactant | | $O_3$ | | | | | | | |
| Chemical vapor deposition material-containing gas flow time (s) | Li-HFIP (4sccm) | - | - | - | - | - | - | - | - |
| | Li-HFIP (1sccm) | 5 | 20 | 5 | 20 | 5 | 20 | 5 | 20 |
| Analysis results | Growth rate (Angstrom/cycle) | 0.11 | 0.07 | 0.24 | 0.18 | 0.27 | 0.19 | 0.28 | 0.12 |
| | Reflection coefficient | 1.35 | 1.21 | 1.23 | 1.36 | 1.32 | 1.31 | 1.32 | 1.26 |

**Claims**

**1.** Chemical vapor deposition material represented by formula (1) below

[Chemical notation 1]

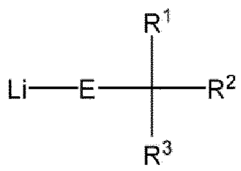

(1)

(here, E is an oxygen atom, sulfur atom, selenium atom or tellurium atom;
$R^1$, $R^2$ and $R^3$ are each independently a hydrogen atom or a C1-7 straight-chain, branched or cyclic organic group,
and at least one of $R^1$, $R^2$ and $R^3$ contains a fluorine atom;
also, $R^1$, $R^2$ and $R^3$ are not all hydrogen atoms).

**2.** The chemical vapor deposition material claimed in Claim 1, where $R^1$, $R^2$ and $R^3$ are each independently a hydrogen atom or a C1-5 fluorinated alkyl group, C2-5 alkenyl group, C2-5 fluorinated alkenyl group or C2-5 fluorinated alkoxyalkyl group.

**3.** The chemical vapor deposition material claimed in Claim 2, where at least one of $R^1$, $R^2$ and $R^3$ is $-CF_3$, $-CF_2CF_3$, $-CF_2CF_2CF_3$, $-(CH_2CH=CH_2)$ or $-(CH_2OCH_2CF_3)$.

**4.** The chemical vapor deposition material claimed in Claim 3, where R1 is $-CF_3$.

**5.** The chemical vapor deposition material claimed in Claim 4, where at least one of $R^2$ and $R^3$ is a hydrogen atom.

**6.** The chemical vapor deposition material claimed in Claim 1, where E is an oxygen atom.

**7.** A film production method provided with a formation step whereby the chemical vapor deposition material claimed in any of Claims 1 to 6 is vapor-deposited onto a substrate to form a film.

**8.** The film production method claimed in Claim 7, where said formation step proceeds in the presence of at least one coreactant chosen from the group consisting of $O_2$, $O_3$, $H_2O$, $H_2O_2$, NO, $NO_2$, $N_2O$, $PH_3$ and TMPO.

**9.** The film production method claimed in Claim 8, where the temperature of the reactor in said formation step is ≥130°C to ≤350°C.

**10.** The film production method claimed in Claim 7, where said film contains a compound represented by $Li_xO_yF_z$ wherein

$$2.6 \leq x \leq 4.3,$$

$$1.8 \leq y \leq 2.3,$$

and

$$4.3 \leq z \leq 6.6.$$

**11.** The film production method claimed in Claim 10, where the reflection coefficient of said film is ≥1.1 to ≤1.5.

**12.** Film that contains a compound represented by $Li_xO_yF_z$ wherein

$$2.6 \leq x \leq 4.3,$$

$$1.8 \leq y \leq 2.3,$$

and

$$4.3 \leq z \leq 6.6.$$

**13.** The film claimed in Claim 12, where the reflection coefficient of said film is ≥1.1 to ≤1.5.

**INTERNATIONAL SEARCH REPORT**

International application No. **PCT/JP2023/020483**

**A. CLASSIFICATION OF SUBJECT MATTER**

***C23C 16/18***(2006.01)i; ***C07C 31/38***(2006.01)i; ***C07F 1/02***(2006.01)i; ***C23C 16/455***(2006.01)i
FI: C23C16/18; C07F1/02; C23C16/455; C07C31/38

According to International Patent Classification (IPC) or to both national classification and IPC

**B. FIELDS SEARCHED**

Minimum documentation searched (classification system followed by classification symbols)

C23C16/18; C07C31/38; C07F1/02; C23C16/455

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | JP 5-289306 A (DAINIPPON PRINTING CO., LTD.) 05 November 1993 (1993-11-05) paragraph [0025] | 12-13 |
| A | | 1-11 |
| A | US 2021/0388010 A1 (SAMSUNG ELECTRONICS CO., LTD.) 16 December 2021 (2021-12-16) paragraphs [0168], [0177], [0231] | 1-13 |

☐ Further documents are listed in the continuation of Box C.   ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **03 August 2023** | **15 August 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2023/020483**

| Patent document cited in search report | Publication date (day/month/year) | Patent family member(s) | Publication date (day/month/year) |
|---|---|---|---|
| JP 5-289306 A | 05 November 1993 | US 5380608 A<br>column 17, lines 17-21<br>KR 10-0249726 B1 | |
| US 2021/0388010 A1 | 16 December 2021 | KR 10-2021-0155744 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2017048445 A